# EUROPEAN PATENT SPECIFICATION

(11) **EP 1 684 807 B1**
(45) Date of publication and mention of the grant of the patent: **02.09.2009**
(21) Application number: 04810280.0
(22) Date of filing: 04.11.2004
(51) Int. Cl.: A61K 49/00

(54) **METHOD OF IDENTIFYING RESPONDERS TO TREATMENT WITH INSULIN SENSITIZERS**
VERFAHREN ZUR IDENTIFIZIERUNG VON RESPONDERN AUF DIE BEHANDLUNG MTI INSULINSENSIBILISATOREN
METHODE D'IDENTIFICATION DE SUJETS REPONDANTS A UN TRAITEMENT AVEC DES AGENTS SENSIBILISANTS A L'INSULINE

(30) Priority: 07.11.2003 US 518390 P
(43) Date of publication of application: 02.08.2006
(73) Proprietor: Merck & Co., Inc., Rahway, New Jersey 07065-0907 (US); ALBERT EINSTEIN COLLEGE OF MEDICINE OF YESHIVA UNIVERSITY, Bronx New York 10461 (US)
(72) Inventor: WAGNER, John, A., Rahway, New Jersey 07065-0907 (US); SCHERER, Philipp, E., Bronx, New York 10461 (US); PAJVANI, Utpal, B., Bronx, New York 10461 (US)
(74) Representative: Buchan, Gavin MacNicol
(86) International application number: PCT/US2004/036648
(87) International publication number: WO 2005/046734

(56) References cited:
- EP-A- 1 681 566
- AU-B2- 2002 328 630
- TSAO TSU-SHUEN ET AL: "Oligomerization state-dependent activation of NF-kappaB signaling pathway by adipocyte complement-related protein of 30 kDa (Acrp30)" JOURNAL OF BIOLOGICAL CHEMISTRY, AMERICAN SOCIETY OF BIOLOCHEMICAL BIOLOGISTS, BIRMINGHAM,, US, vol. 277, no. 33, 16 August 2002 (2002-08-16), pages 29359-29362, XP002424896 ISSN: 0021-9258
- PAJVANI U B ET AL: "Structure-function studies of the adipocyte-secreted hormone Acrp30/adiponectin. Implications for metabolic regulation and bioactivity" JOURNAL OF BIOLOGICAL CHEMISTRY, AMERICAN SOCIETY OF BIOLOCHEMICAL BIOLOGISTS, BIRMINGHAM,, US, vol. 278, no. 11, 14 March 2003 (2003-03-14), pages 9073-9085, XP002251775 ISSN: 0021-9258
- WAKI H ET AL: "Impaired Multimerization of Human Adiponectin Mutants Associated with Diabetes" JOURNAL OF BIOLOGICAL CHEMISTRY, AMERICAN SOCIETY OF BIOLOCHEMICAL BIOLOGISTS, BIRMINGHAM,, US, vol. 278, no. 41, 10 October 2003 (2003-10-10), pages 40352-40363, XP002988190 ISSN: 0021-9258
- IWAKI M. ET AL: 'Induction of Adiponectin, a Fat-Derived Antidiabetic and Antiatherogenic Factor, by Nuclear Receptors' DIABETES vol. 52, July 2003, pages 1655 - 1663, XP002986548

## Description

### BACKGROUND OF THE INVENTION

Adipocytes can influence whole-body metabolism through modulation of systemic free fatty acid levels and through secretion of adipocyte-specific or -enriched proteins collectively known as adipokines. Recent publications have underscored the importance of adipocyte-secreted molecules in energy homeostasis and metabolism (1-3). Adipokines such as leptin, resistin, adiponectin (also known as Acrp30, AdipoQ, aPM1 and GBP28), adipsin, interleukin-6, plasminogen activator inhibitor-1 and many more have been shown to affect systemic insulin action, carbohydrate and lipid metabolism (4). Some of these adipokines have synergistic effects while others, such as resistin and adiponectin, have competing effects; pharmacological doses of resistin deactivate the repressive effects of insulin on gluconeogenesis (5), whereas adiponectin increases insulin sensitivity, leading to enhanced inhibition of hepatic glucose output (6). Furthermore, the human adiponectin gene lies on chromosome 3 (3q27) and a recent genome-wide scan for phenotypes related to the obesity-metabolic syndrome revealed this region of chromosome 3 to be a novel diabetes susceptibility locus (reviewed in (7)). Since then, several groups have hypothesized and provided evidence that genetic variability within the adiponectin gene leads to alteration of serum levels of the protein, and generally predisposes patients to insulin resistance. Decreased serum adiponectin is now considered a feature of obesity and typically correlates with lowered indices of insulin sensitivity (8, 9). Several studies have suggested that the decrease in serum adiponectin levels is a contributing factor and not merely a result of declining insulin sensitivity. Genetic and pharmacological data support a direct impact of the protein on insulin sensitivity (2, 10, 11).

The potential importance of adiponectin as a therapeutic target is underscored by the dramatic upregulation of this adipokine in response to treatment with the antidiabetic, insulin-sensitizing agents known as thiazolidinediones (TZDs) (12-14). TZDs are active in many animal models of genetic or acquired insulin resistance, suggesting that these drugs improve insulin sensitivity regardless of the underlying cause (15). Clinical studies confirmed the insulin sensitizing effects of TZDs in type 2 diabetic patients in whom these drugs lower both fasting and postprandial glucose and insulin levels. However, the molecular mechanisms of TZD action are still not fully understood. TZDs function as exogenous ligands for PPARγ, a transcription factor highly expressed in adipocytes, but which is also found at lower levels in other tissues (16). The high level of expression of the primary TZD target (PPARγ) in adipose tissue suggests that adipocytes may play a critical role in mediating at least some aspects of TZD action. This hypothesis has been further supported by studies of "fatless" mice that displayed reduced metabolic improvement in response to TZD treatment (17); although TZD treatment effectively lowered serum triglycerides, measures of insulin sensitivity were unaffected in this animal model lacking significant adipose tissue. A number of studies have demonstrated that in mice and humans, TZD treatment effects transcriptional upregulation accompanied by increased production and secretion of adiponectin from adipocytes (2, 12-14). Interestingly, a significant increase in circulating adiponectin preceded decreased serum glucose and triglyceride levels achieved with TZD treatment of a *db*/*db* mouse cohort (12). There is still ongoing discussion among researchers in the field as to whether the TZD-mediated induction of adiponectin is causative or simply diagnostic of improving insulin sensitivity. A causative role has been challenged by the report that discordance exists between improvements in insulin sensitivity and induction in adiponectin (13). Although the vast majority of patients induce adiponectin expression and secretion in response to TZD treatment (albeit, to various degrees), only 50-70% of patients demonstrate clinically improved insulin sensitivity (reviewed in (18)). This suggests that induction of adiponectin in any particular individual is neither predictive nor correlative to quantitative improvements in insulin sensitivity.

It was recently demonstrated that adiponectin exists in at least two forms in serum, as a trimer-dimer referred to as a low molecular weight (LMW) complex and as a high molecular weight (HMW) complex consisting of 12-18 subunits (19). These oligomeric complexes are stable both *in vitro* and *in vivo* and can readily be resolved by velocity sedimentation or gel filtration chromatography. They are differentially regulated by various metabolic stimuli. Upon insulin treatment in rodents or humans, serum adiponectin levels decrease (13). In mice it has been shown that this is the result of a specific decrease in circulating HMW complexes (19). Similarly, an oral glucose challenge will result in a selective disappearance of the HMW from serum. The importance of HMW and of the ratio between these two oligomeric forms (HMW to LMW), rather than the absolute amounts, has not been recognized as important in determining or controlling insulin sensitivity.

Pioglitazone and rosiglitazone are PPAR gamma agonists that contain a benzyl thiazolidinedione (TZD) unit as part of their structure. These compounds reduce insulin resistance in 50-70% of diabetic patients to whom it is administered, and thereby ameliorate the symptoms of type 2 diabetes in these patients. Patients who demonstrate clinically improved insulin sensitivity are referred to as "responders" to treatment. Patients who do not demonstrate clinically improved insulin sensitivity are "non-responders."

Several months of treatment with a TZD or other insulin sensitizer are required before a patient can be identified as a responder or non-responder based on clinical response and improvement in hemoglobin A1C. It would be advantageous to identify patients who are likely to be non-responders to treatment with a TZD or other insulin sensitizer in a shorter period of time (e.g. 1-4 weeks) so that an alternative treatment regimen can be initiated sooner.

A method of identifying responders and non-responders in a shorter time period is disclosed herein. Responders to treatment with TZD's or other insulin sensitizers will be readily identified within four weeks, and preferably within two weeks, and even more preferably within one week of the start of treatment, by following the methods described herein. The method is based on the measurement of the amount of adiponectin, including HMW and LMW adiponectin, in the patient's serum or plasma.

AU 2002328630 describes a method of monitoring carbohydratic metabolism disorders by assaying adiponectin. Tsao et al (J. Biol. Chem., 277, 29359 (2002), and Waki et al (J. Biol. Chem., 278, 40352 (2004)) describes the role of adiponectin multimers in diabetes.

### SUMMARY OF THE INVENTION

The present application is about methods as described in the appended claims. A patient who is a responder to a therapeutic treatment for insulin resistance or for one or more diseases associated with type 2 diabetes can be identified by the following method, which comprises the steps of:
Measuring the amount of HMW adiponectin and the amount of total adiponectin or LMW adiponectin in plasma or serum obtained from the patient before the commencement of said therapeutic treatment; and
Measuring the amount of HMW adiponectin and the amount of total adiponectin or LMW adiponectin in plasma or serum obtained from said patient one or more times after the commencement of said therapeutic treatment, wherein said patient is determined to be a responder to said therapeutic treatment if the ratio of the amount of HMW adiponectin to the amount of total adiponectin or LMW adiponectin increases after said therapeutic treatment commences.

The therapeutic treatment generally comprises the step of administering an effective amount of one or more insulin sensitizing pharmaceuticals, such as a thiazolidinedione (also referred to as a TZD); a PPAR gamma agonist that is not a TZD; or an insulin sensitizing compound that works by a different mechanism than PPAR gamma agonism. PPAR gamma agonists that have additional therapeutic activities in addition to PPAR gamma agonism, such as PPAR alpha gamma dual agonists, may also be tested by the methods used herein to determine whether the patient is a responder to treatment with the PPAR gamma agonist. The method may also be applicable to patients being treated with PPAR gamma partial agonists, also known as selective PPAR gamma modulators (SPPARM's), PPAR alpha-gamma dual partial agonists (selective PPAR alpha-gamma dual selective modulators), and PPAR pan-agonists.

PPAR gamma agonists that have a TZD structure include pioglitazone, rosiglitazone, ciglitazone, darglitazone, englitazone, balaglitazone, isaglitazone, troglitazone, netoglitazone, MCC-555, and BRL-49653. Other PPAR gamma agonists, some of which have a TZD structure, include CLX-0921, 5-BTZD, GW-0207, LG-100641, LY-300512, NN-2344, LY-818, GW-677954, GW-7282, and T-131. Preferred PPAR gamma agonists include rosiglitazone and piogitazone.

PPAR alpha/gamma dual agonists exhibit both alpha and gamma agonism and may be used to concurrently treat type 2 diabetes and to reduce lipids. PPAR alpha/gamma agonists include KRP-297 (MK-0767), muraglitazar (BMS-298585), farglitazar, ragaglitazar, tesaglitazar (AZ-242), JT-501, GW-2570, GI-262579, CLX-0940, GW-1536, GW1929, GW-2433, L-796449, LR-90, SB-219994, LY-578, LY-4655608, LSN-862, LY-510929, and LY-929. Preferred PPAR alpha/gamma agonists include KRP-297 (MK-0767), muraglitazar (BMS-298585), farglitazar, and tesaglitazar (AZ-242).

The method disclosed herein is also expected to be effective in determining whether a patient is likely to be a responder to treatment with a TZD or non-TZD PPAR gamma agonist when the TZD or non-TZD PPAR gamma agonist is used in combination (e.g. fixed combination) or concomitantly with another drug or drugs that may be used to treat type 2 diabetes or insulin resistance. Such other drug is for example a biguanide (e.g. metformin); a sulfonylurea; another chemical class of insulin secretagogue other than a sulfonylurea, such as a meglitinide; insulin (which may be formulated for subcutaneous or intramuscular injection, or in a formulation for avoiding the need for injection, such as oral, buccal, or nasal); a DP-IV inhibitor; a PTP-1B inhibitor; a GLP-1 analog; a glycogen phosphorylase inhibitor; a glucagon receptor antagonist; a hydroxysterol dehydrogenase (HSD-1) inhibitor; a glucokinase activator; or is from another class of anti-diabetic compounds. The method disclosed herein is also expected to be effective in determining whether a patient is likely to be a responder to treatment with a TZD or non-TZD PPAR gamma agonist when the TZD or non-TZD PPAR gamma agonist is administered in combination (fixed combination) or concomitantly with another drug or drugs that may be used to treat obesity in an obese patient who also has type 2 diabetes or insulin resistance. Such other drug is for example sibutramine, orlistat, phentermine, an Mc4r agonist, cannabinoid receptor 1 (CB-1) antagonist/inverse agonist, a β₃ adrenergic agonist, or a drug from another class of anti-obesity compounds. The method is also expected to be effective in determining whether a patient is a responder to treatment with a TZD or non-TZD PPAR agonist when it is administered concomitantly or in a fixed combination with one or more drugs used to reduce total cholesterol or LDL-cholesterol and/or raise HDL-cholesterol, such as an HMG-CoA reductase inhibitor (lovastatin, simvastatin, rosuvastatin, pravastatin, fluvastatin, atorvastatin, rivastatin, pitavastatin, ZD-4522, and other statins); niacin; a cholesterol absorption inhibitor (ezetimibe); a CETP inhibitor (torcetrapib); a PPAR alpha agonist (fenofibrate, gemfibrizol, clofibrate, or bezafibrate); an ACAT inhibitor (avasimibe); an anti-oxidant (probucol); or a bile acid sequestrant (cholestyramine).

The preferred analysis is to measure and compare the ratio of the amount of HMW adiponectin to the amount of total adiponectin in the patient's plasma or serum before treatment begins and then after treatment has proceeded for a time long enough for the changes in the ratio of the amount of HMW and the amount of total adiponectin to reflect whether the patient will respond to treatment. This ratio is defined herein as S_{A}, which is the calculated ratio of HMW/(HMW+LMW). Changes in this ratio of HMW to total HMW + LMW adiponection are the best predictors of whether a patient will respond positively to treatment with an insulin sensitizer. After treatment has proceeded, a patient who is a likely responder to therapeutic treatment will have a ratio of the amount of HMW adiponectin to the amount of total adiponectin that has increased during treatment. Likely increases in this ratio that are indicative of a positive response may be for example 20%, 25%, 30%, 40%, 50% and 75%. These increases will be observed within four weeks after treatment commences, preferably within two weeks after treatment commences, and most preferably within one week after treatment commences.

### BRIEF DESCRIPTION OF THE DRAWINGS

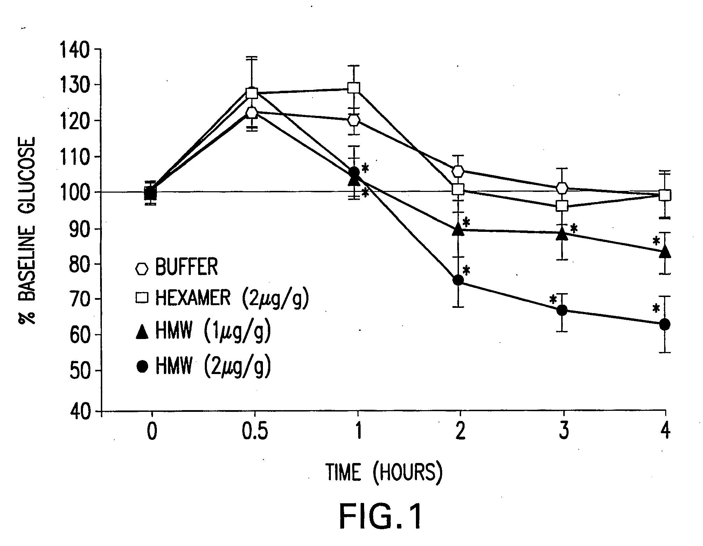

**Figure 1** illustrates that intravenous injection of HMW, but not hexameric (LMW) adiponectin, leads to a dose-dependent decrease in serum glucose. The figure compares changes in serum glucose of male adiponectin knockout mice injected with HMW adiponectin (1 or 2 µg/g body weight), LMW adiponectin (2 µg/g body weight), or buffer.

### DETAILED DESCRIPTION OF THE INVENTION

Adiponectin is an adipocyte-specific secretory protein that circulates in serum as a hexamer of relatively low molecular weight (LMW) and a larger multimeric structure of high molecular weight (HMW). Serum levels of the protein correlate with systemic insulin sensitivity. The full-length protein affects hepatic gluconeogenesis through improved insulin sensitivity, and a proteolytic fragment of adiponectin stimulates β oxidation in muscle. It has been found that the ratio, and not the absolute amounts, between these two oligomeric forms (HMW to LMW) is critical in determining insulin sensitivity. A new index, S_{A}, is defined as the ratio of HMW/(HMW+LMW). In *db*/*db* mice, the values of S_{A} are lower than in wildtype littermates, despite similar total adiponectin levels. Furthermore, S_{A} increases with PPARγ agonist treatment (TZD). Changes in S_{A} serve as a quantitative indicator of improvements in insulin sensitivity obtained during TZD treatment, whereas changes in total serum adiponectin levels do not correlate well at the individual level.

### Materials and Methods

*Velocity sedimentation*/*gel filtration chromatography for separation of adiponectin complexes -* 5-20% sucrose gradients in 10mM HEPES pH 8, 125mM NaCl were poured stepwise (5%, 10%, 15%, 20%) in 2-ml thin-walled ultracentrifuge tubes (Becton Dickinson) and allowed to equilibrate overnight at 4°C. Following layering of the sample on top (diluted 1:10 with 10mM HEPES pH 8, 125 mM NaCl in the case of serum), the gradients were spun at 55,000 RPM for four hours at 4°C in a TLS55 rotor in a Beckman TL-100 tabletop ultracentrifuge. 150 µL gradient fractions were sequentially retrieved from the top of the gradient and analyzed by quantitative Western blot analysis.

*Immunoblotting-* Separation of proteins by SDS-PAGE, fluorography, and immunoblotting were performed as described previously (20). Primary and secondary antibodies were diluted in TBS with 0.05% Tween-20 and 1% BSA. Horseradish peroxidase conjugated secondary antibodies were detected with enhanced chemiluminescence according to the manufacturer's instructions (Pierce). For quantitative Western blotting, proteins were transferred to BA83 nitrocellulose (Schleicher & Schuell) after SDS-PAGE. Nitrocellulose membranes were stained with Ponceau S solution to ensure even and complete transfer of all samples and subsequently blocked in TBS with 0.05% Tween-20 and 5% non-fat dry milk. An affinity-purified rabbit anti-mouse adiponectin antibody raised against a peptide comprising the hypervariable region (EDDVTTTEELAPALV) was used; this antibody recognizes a single band by Western blot analysis that can effectively be competed with excess immune peptide. For the analysis of human serum samples, a rabbit anti-human adiponectin antibody, directed against the hypervariable region of the human protein (DQETTTQGPGV), was employed. Both primary antibodies were visualized with an ¹²⁵I-derivatized secondary goat anti-rabbit antibody (Amersham). Blots were analyzed with a Phosphoimager (Molecular Dynamics) and fractions 4-6 and 9-11 from velocity sedimentation (LMW and HMW adiponectin, respectively) were quantitated with Imagequant Software.

*In Vivo Animal Studies -* Male *dbldb* mice and control mice (Lepr^{db}+/Lepr^{db}+ and Lepr^{db}+/+m, respectively, Jackson Labs) were housed 5/cage and allowed *ad lib* access to ground Purina rodent chow 5001 and water. The animals, and their food, were weighed every 3 days and were dosed daily by gavage with vehicle (0.25% carboxymethylcellulose) ± 10 mg/kg-day rosiglitazone for 11 days or 10 mg/kg-day PPARα agonist for 7 days (compound 10, (21)). Plasma adiponectin, glucose and triglyceride levels were determined from blood obtained by tail bleeds at 3-4 day intervals during the studies. Wildtype animals (C57/B16J) used in adipose extraction and adiponectin knockout animals for *in* vivo adiponectin activity studies were maintained in the same manner. All animal protocols were approved by the Albert Einstein Animal Committee.

### Human Clinical Study Protocol - Study A

This was a single center, double-blind, randomized, placebo-controlled, parallel group study with treatments including placebo and rosiglitazone (4 mg bid) for 14 days. Twenty nondiabetic subjects were treated in this analysis (n=10/group). Plasma for adiponectin concentration determination was obtained predose on Day 1 (baseline) and 2 hours after the last dose on Day 14. All 20 subjects were healthy males who varied in age from 18 to 42 years (mean age 24 years) and in weight from 61 to 110 kg (mean weight 89 kg). These subjects refrained from all other medication use from 14 days prior to completion of the trial. They had no evidence or family history of diabetes mellitus, baseline fasting plasma glucose was < 110 mg/dL, and baseline fasting plasma lipid profile (including triglycerides and total cholesterol) was within the reference range for the laboratory. All subjects gave written informed consent and the clinical protocol was reviewed by and approved by Commissie voor Medische Ethiek, Antwerp, Belgium.

### Results of Animal Studies and Study A

*Diabetic mice display decreased HMW*/*total adiponectin ratio despite comparable levels of total serum adiponectin.* While adiponectin levels are significantly reduced in states of decreased insulin sensitivity in humans under essentially all circumstances, insulin resistance in mice is often but not always associated with reduced adiponectin levels. This is particularly relevant for monogenic lesions such as the ones found in *dbldb* and *oblob* mice. It was previously shown that *dbldb* mice demonstrate levels of circulating adiponectin that are comparable with lean heterozygote littermates (12). To determine whether differences between these animals can be explained (at least partially) on the basis of differential distribution of adiponectin complexes in serum, we analyzed serum from male *dbldb* and *dbl+* mice by velocity sedimentation followed by SDS-PAGE. Similar to previous findings, lean and obese animals had comparable total levels of adiponectin circulating in serum. However, db/db mice exhibited a significantly decreased percentage of adiponectin in the HMW form. Similar reductions in %HMW adiponectin can be seen in a number of other diabetic mouse models, including the *ob*/*ob* mouse (not shown).

*Thiazolidinedione treatment affects circulating HMW*/*LMW adiponectin complex ratios in mice and humans.* Thiazolidinedione (TZD) treatment leads to an induction of serum adiponectin and ameliorates the hyperglycemia, hypertriglyceridemia and insulin resistance in the *db*/*db* mouse model within an 11-day course of treatment (12). To determine if TZD treatment affects the relative circulating concentrations of adiponectin oligomers in serum, a cohort of male *db*/*db* mice was treated with rosiglitazone, and adiponectin complexes were analyzed by velocity sedimentation. Prior to treatment, adiponectin is predominantly found in the LMW (hexameric) form of adiponectin, consistent with values from wildtype male mice. However, following 11 days of rosiglitazone treatment, the percentage of adiponectin found in the high molecular weight (HMW) form, nearly doubled, to approximately 45% of total circulating adiponectin. Placebo treatment did not result in any significant change in adiponectin oligomeric distribution (not shown), nor did a 7-day treatment with PPARα agonist that was equally successful in reducing serum glucose, triglyceride and insulin levels (by 45%, 45% and 80% respectively). This indicates that this shift in complex distribution can be attributed directly to TZD treatment and is not an indirect consequence of a systemic improvement of metabolic parameters.

In order to see if this relative increase in HMW adiponectin can also be observed in human subjects treated with TZDs, the effects in a cohort of non-diabetic human males was tested ("Study A"; (12)). They received two weeks of treatment with either rosiglitazone or placebo, and adiponectin complexes were analyzed in a double-blind fashion by velocity sedimentation pre- and post-treatment. Only minor changes in total circulating adiponectin levels or in either HMW or LMW adiponectin complex were seen in placebo-treated patients. By comparison, rosiglitazone-treated patients demonstrated significantly increased total adiponectin (about 2-fold higher than placebo). The increase in total adiponcetin was primarily the result of a dramatic increase in the circulating HMW form. As a consequence, the HMW/total adiponectin ratio was significantly increased in rosiglitazone treated individuals, with the post-treatment value being about 45-50%, which is about double the pre-treatment value of 20-25%.

*Intravenous injection of HMW adiponectin, but not hexameric (LMW) adioponectin, leads to decreased serum glucose in mice.* Previous work has demonstrated that properly folded and assembled full-length adiponectin, when introduced into animals through either intraperitoneal or intravenous injection, leads to a significant decrease in serum glucose levels. To determine if there is any evidence for differential biochemical activity of the HMW and LMW adiponectin complexes, purified HMW (1 or 2µg/g body weight) or LMW (2µg/g body weight) adiponectin was injected into male animals. To avoid any confounding effects of various circulating endogenous complexes, these injections were performed in mice carrying a chromosomal deletion at the adiponectin locus, so that the mice completely lacked any endogenous circulating adiponectin.

The data are presented in **Figure 1****.** Male adiponectin knockout animals of 10-12 weeks of age were injected via tail vein with 2 µg/g body weight HMW adiponectin (n=6) (solid circles), 2 µg/g LMW adiponectin (n=6) (open squares), 1 µg/g HMW adiponectin (n=6) (solid triangles) or buffer (n=6) (open circles). Serum glucose was assayed by glucometer at various time points post-injection. Starting glucose levels, arbitrarily set to 100% for each cohort, averaged 150±5 mg/dl across all cohorts. Changes in glucose are plotted as a % of baseline (starting) glucose against time (hours) after injection. Values that significantly differ from the buffer control are indicated by an asterisk (p<0.05). The plots in **Figure 1** illustrate that HMW adiponectin dose-dependently reduced plasma glucose levels, whereas purified hexameric (LMW) adiponectin lacked the ability to induce a decrease in plasma glucose levels compared to injection of buffer. Since male mice typically display about 80% of their adiponectin in the LMW form (corresponding to a 12- to 15-fold molar excess), solubility issues with respect to the purified complexes prevented the injection of mixtures of the two complexes that would effectively mimic this extreme molar excess of LMW complexes.

*Adiponectin complex secretion is regulated at the level of adipose tissue.* It was previously shown that iodinated adiponectin complexes are stable in serum and do not interconvert post-secretion. These observations were recently confirmed in adiponectin knockout animals using non-derivatized, fully native adiponectin complexes (data not shown). This supports the hypothesis that the mechanism of increased HMW adiponectin post-TZD treatment is mediated by adipocytes, through differential secretion of the two oligomeric forms. Various adipose tissues and serum from male and female mice were analyzed by velocity sedimentation to determine the complex distribution from these animals. As previously reported, male and female mice display differential levels of adiponectin complexes in serum, with male animals displaying about 25% of their serum adiponectin in the HMW form, while female mice have slightly more than double that percentage (~50% HMW). Surprisingly, both males and females have similar proportions of HMW adiponectin within their adipose tissue - between 70-90% of adiponectin associated with adipose tissue is in the HMW form, in sharp contrast to the serum distribution within the same mice. The differences between tissue-associated and serum adiponectin ratios were quantitated and are particularly striking for male mice, although significant increases in HMW adiponectin in adipose tissue is observed in mice of both genders. A similar pattern is observed with human serum and adipose tissue.

### References

*1.* Zhang, Y., Proenca, R., Maffel, M., Barone, M., Leopold, L., and Friedman, J.M. 1994. Posional cloning of the mouse obese gene and its human homologue. Nature 372:425-432*.*
*2.* Berg, A.H., Combs, T., Du, X., Brownlee, M., and Scherer, P.E. 2001. The Adipocyte-Secreted Protein Acrp30 Enhances Hepatic Insulin Action. Nat Med 7:947-953*.*
*3.* Steppan, C.M., Bailey, S.T., Bhat, S., Brown, E.J., Banerjee, R.R., Wright, C.M., Patel, H.R., Ahima, R.S., and Lazar, M.A. 2001. The hormone resistin links obesity to diabetes. Nature 409:307-312*.*
*4.* Lyon, C.J., Law, R.E., and Hsueh, W.A. 2003. Minireview: adiposity, inflammation, and atherogenesis. Endocrinology 144:2195-2200*.*
*5.* Rajala, M.W., Obici, S., Scherer, P.E., and Rossetti, L. 2003. Adipose-derived resistin and gut-derived resistin-like molecule-beta selectively impair insulin action on glucose production. J Clin Invest 111:225-230*.*
*6.* Combs, T.P., Berg, A.H., Obici, S., Scherer, P.E., and Rossetti, L. 2001. Endogenous Glucose Production is Inhibited by the Adipose-Derived Protein Acrp30. J. Clin. Inv. 108:1875-1881*.*
*7.* Vasseur, F., Lepretre, F., Lacquemant, C., and Froguel, P. 2003. The genetics of adiponectin. Curr Diab Rep 3:151-158*.*
*8.* Weyer, C., Funahashi, T., Tanaka, S., Hotta, K., Matsuzawa, Y., Pratley, R.E., and Tataranni, P.A. 2001. Hypoadiponectinemia in obesity and type 2 diabetes: close association with insulin resistance and hyperinsulinemia. J Clin Endocrinol Metab 86:1930-1935*.*
*9.* Cnop, M., Havel, P.J., Utzschneider, K.M., Carr, D.B., Sinha, M.K., Boyko, E.J., Retzlaff, B.M., Knopp, R.H., Brunzell, J.D., and Kahn, S.E. 2003. Relationship of adiponectin to body fat distribution, insulin sensitivity and plasma lipoproteins: evidence for independent roles of age and sex. Diabetologia 46:459-469*.*
*10.* Yamauchi, T., Kamon, J., Waki, H., Terauchi, Y., Kubota, N., Hara, K., Mori, Y., Ide, T., Murakami, K., Tsuboyama-Kasaoka, N., et al. 2001. The fat-derived hormone adiponectin reverses insulin resistance associated with both lipoatrophy and obesity. Nat. Med. 7:941-946*.*
*11.* Fruebis, J., Tsao, T.S., Javorschi, S., Ebbets-Reed, D., Erickson, M.R., Yen, F.T., Bihain, B.E., and Lodish, H.F. 2001. Proteolytic cleavage product of 30-kDa adipocyte complement-related protein increases fatty acid oxidation in muscle and causes weight loss in mice. Proc Natl Acad Sci U S A 98:2005-2010*.*
*12.* Combs, T.P., Wagner, J.A., Berger, J., Doebber, T., Wang, W.J., Zhang, B.B., Tanen, M., Berg, A.H., O'Rahilly, S., Savage, D.B., et al. 2002. Induction of adipocyte complement-related protein of 30 kilodaltons by PPARgamma agonists: a potential mechanism of insulin sensitization. Endocrinology 143:998-1007*.*
*13.* Yu, J.G., Javorschi, S., Hevener, A.L., Kruszynska, Y.T., Norman, R.A., Sinha, M., and Olefsky, J.M. 2002. The effect of thiazolidinediones on plasma adiponectin levels in normal, obese, and type 2 diabetic subjects. Diabetes 51:2968-2974*.*
*14.* Maeda, N., Takahashi, M., Funahashi, T., Kihara, S., Nishizawa, H., Kishida, K., Nagaretani, H., Matsuda, M., Komuro, R., Ouchi, N., et al. 2001. PPARgamma ligands increase expression and plasma concentrations of adiponectin, an adipose-derived protein. Diabetes 50: 2094-2099*.*
*15.* Day, C. 1999, Thiazolidinediones: a new class of antidiabetic drugs. Diabet Med 16:179-192*.*
*16.* Lehmann, J.M., Moore, L.B., Smith-Oliver, T.A., Wilkison, W.O., Willson, T.M., and Kliewer, S.A. 1995. An antidiabetic thiazolidinedione is a high affinity ligand for peroxisome proliferator-activated receptor gamma (PPAR gamma). J Biol Chem 270:12953-12956*.*
*17.* Chao, L., Marcus-Samuels, B., Mason, M.M., Moitra, J., Vinson, C., Arioglu, E., Gavrilova, O., and Reitman, M.L. 2000. Adipose tissue is required for the antidiabetic, but not for the hypolipidemic, effect of thiazolidinediones. J Clin Invest 106:1221-1228*.*
*18.* Olefsky, J.M. 2000. Treatment of insulin resistance with peroxisome proliferator-activated receptor gamma agonists. J Clin Invest 106:467-472*.*
*19.* Pajvani, U.B., Du, X., Combs, T.P., Berg, A.H., Rajala, M.W., Schulthess, T., Engel, J., Brownlee, M., and Scherer, P.E. 2003. Structure-function studies of the adipocyte-secreted hormone Acrp30/adiponectin: Implications for metabolic regulation and bioactivity. J Biol Chem 278:9073-9085*.*
*20.* Scherer, P.E., Lisanti., M.P., Baldini, G., Sargiacomo, M., Corley-Mastick, C., and Lodish, H.F. 1994. Induction of Caveolin during Adipogenesis and Association of GLUT4 with Caveolin-rich vesicles. J Cell Biol 127:1233-1243*.*
*21.* Adam, A.D., Hu, Z., von Langen, D., Dadiz, A., Elbrecht, A., MacNaul, K.L., Berger, J.P., Zhou, G., Doebber, T.W., Meurer, R., et al. 2003. O-Arylmandelic acids as highly selective human PPARalpha/gamma agonists. Bioorg Med Chem Lett in press*.*

## Claims

1. A method of determining whether an insulin resistant patient is a responder to a therapeutic treatment for insulin resistance, comprising the.steps of:
Measuring the amount of HMW adiponectin and the amount of total adiponectin or LMW adiponectin in plasma or serum obtained from the patient before the commencement of said therapeutic treatment; and
Measuring the amount of HMW adiponectin and the amount of total adiponectin or LMW adiponectin in plasma or serum obtained from said patient one or more times after the commencement of said therapeutic treatment,
wherein said patient is determined to be a responder to said therapeutic treatment if the ratio of the amount of HMW adiponectin to the amount of total adiponectin or LMW adiponectin increases after said therapeutic treatment commences.

2. The method of Claim 1, wherein said therapeutic treatment has comprised the administration of an effective amount of one or more insulin sensitizing pharmaceuticals.

3. The method of Claim 2, wherein said patient is determined to be a responder to said therapeutic treatment if the ratio of the amount of HMW adiponectin to the amount of total adiponectin or LMW adiponectin increases by at least 20% after said therapeutic treatment commences.

4. The method of Claim 2, wherein said patient is determined to be a responder to said therapeutic treatment if the ratio of the amount of HMW adiponectin to the amount of total adiponectin or LMW adiponectin increases by at least 25% after said therapeutic treatment commences.

5. The method of Claim 2, wherein said patient is determined to be a responder to said therapeutic treatment if the ratio of the amount of HMW adiponectin to the amount of total adiponectin or LMW adiponectin increases by at least 30% after said therapeutic treatment commences.

6. The method of Claim 2, wherein said patient is determined to be a responder to said therapeutic treatment if the ratio of the amount of HMW adiponectin to the amount of total adiponectin or LMW adiponectin increases by at least 40% after said therapeutic treatment commences.

7. The method of Claim 2, wherein said patient is determined to be a responder to said therapeutic treatment if the ratio of the amount of HMW adiponectin to the amount of total adiponectin or LMW adiponectin increases by at least 50% after said therapeutic treatment commences.

8. The method of Claim 2, wherein said patient is determined to be a responder to said therapeutic treatment if the ratio of the amount of HMW adiponectin to the amount of total adiponectin or LMW adiponectin increases by at least 75% after said therapeutic treatment commences.

9. The method of Claim 2, wherein said insulin sensitizing pharmaceuticals are selected from the group consisting of PPAR-gamma agonists, PPAR-gamma partial agonists, and PPAR alpha-gamma dual agonists.

10. The method of Claim 2, wherein said insulin sensitizing pharmaceutical has a TZD group in its structure.

11. The method of Claim 2, wherein said insulin sensitizing pharmaceutical is selected from the group consisting of pioglitazone, rosiglitazone, MCC-555, balaglitazone, isaglitazone, netoglitazone, KRP-297 (MK-0767), farglitazar, tesaglitazar (AZ-242), and muraglitazar (BMS-298585).

12. A method according to any previous claim wherein the ratio of the amount of HMW adiponectin to the amount of total adiponectin is measured.

13. The method of Claim 2, wherein said patient is determined to be a responder to said therapeutic treatment if the ratio of the amount of HMW adiponectin to the amount of total adiponectin in said patient's plasma or serum increases by at least 20% within four weeks after said therapeutic treatment commences.

14. The method of Claim 13, wherein said patient is determined to be a responder to said therapeutic treatment if the ratio of the amount of HMW adiponectin to the amount of total adiponectin in said patient's plasma or serum increases by at least 20% within two weeks after said therapeutic treatment commences.

15. The method of Claim 13, wherein said patient is determined to be a responder to said therapeutic treatment if the ratio of the amount of HMW adiponectin to the amount of total adiponectin in said patient's plasma or serum increases by at least 20% within one week after said therapeutic treatment commences.

16. A method of predicting whether a therapeutic treatment for insulin resistance will be effective in ameliorating one or more diseases associated with insulin resistance in a patient in need of treatment for said disease or diseases, comprising the steps of:
Measuring the amount of HMW adiponectin and the amount of total adiponectin in plasma or serum obtained from said patient before commencing said therapeutic treatment; and
Measuring the amount of HMW adiponectin and the amount of total adiponectin in plasma or serum obtained from said patient one or more times after the commencement of said therapeutic treatment;
wherein said therapeutic treatment has comprised the administration of an effective amount of one or more insulin sensitizing pharmaceuticals,
and wherein said therapeutic treatment is predicted to be effective in ameliorating said one or more diseases in said patient if the ratio of the amount of HMW adiponectin to the amount of total adiponectin increases by at least 20% within four weeks after said therapeutic treatment commences.

17. The method according to claim 16 wherein said one or more diseases associated with insulin resistance is selected from the group consisting of Type 2 diabetes, obesity, hypertension, and dyslipidemia.

18. The method of Claim 16, wherein said method is used to predict whether a therapeutic treatment for insulin resistance will be effective in ameliorating hyperglycemia or dyslipidemia in a type 2 diabetic patient.

19. The method of Claim 16, wherein said method is used to predict whether a therapeutic treatment for insulin resistance will be effective in reducing the risk that a non-diabetic patient having impaired glucose tolerance or elevated fasting plasma glucose will develop type 2 diabetes.

20. The method of Claim 16 , wherein said method is used to predict whether a therapeutic treatment for insulin resistance will be effective in ameliorating three or more symptoms of the metabolic syndrome, said symptoms being selected from the group consisting of abdominal obesity, hypertriglyceridemia, low HDL, high blood pressure, and elevated fasting glucose.

21. A method according to any of claims 18-20 wherein said therapeutic treatment is predicted to be effective if the ratio of the amount of HMW adiponectin to the amount of total adiponectin in said patient's plasma or serum increases by at least 25% within four weeks after said therapeutic treatment commences.

22. A method according to any of claims 18-20 wherein said therapeutic treatment is predicted to be effective if the ratio of the amount of HMW adiponectin to the amount of total adiponectin in said patient's plasma or serum increases by at least 30% within four weeks after said therapeutic treatment commences.

23. A method according to any of claims 18-20 wherein said therapeutic treatment is predicted to be effective if the ratio of the amount of HMW adiponectin to the amount of total adiponectin in said patient's plasma or serum increases by at least 40% within four weeks after said therapeutic treatment commences.

24. A method according to any of claims 18-20 wherein said therapeutic treatment is predicted to be effective if the ratio of the amount of HMW adiponectin to the amount of total adiponectin in said patient's plasma or serum increases by at least 50% within four weeks after said therapeutic treatment commences.

25. A method according to any of claims 18-20 wherein said therapeutic treatment is predicted to be effective if the ratio of the amount of HMW adiponectin to the amount of total adiponectin in said patient's plasma or serum increases by at least 75% within four weeks after said therapeutic treatment commences.

## Patentansprüche

1. Ein Verfahren zur Bestimmung, ob ein insulinresistenter Patient ein Responder auf eine therapeutische Behandlung gegen Insulinresistenz ist, umfassend die Schritte:
Messen der Menge an HMW-Adiponektin und der Menge an Gesamt-Adiponektin oder LMW-Adiponektin in Plasma oder Serum, das von dem Patienten vor dem Beginn der therapeutischen Behandlung erhalten wird, und
Messen der Menge an HMW-Adiponektin und der Menge an Gesamt-Adiponektin oder LMW-Adiponektin in Plasma oder Serum, das von dem Patienten ein- oder mehrmals nach dem Beginn der therapeutischen Behandlung erhalten wird,
wobei der Patient als ein Responder auf die therapeutische Behandlung eingestuft wird, wenn das Verhältnis der Menge an HMW-Adiponektin zu der Menge an Gesamt-Adiponektin oder LMW-Adiponektin nach dem Beginn der therapeutischen Behandlung ansteigt.

2. Das Verfahren nach Anspruch 1, bei dem die therapeutische Behandlung die Verabreichung einer wirksamen Menge von einem oder mehreren insulinsensibilisierenden Pharmazeutika umfasst hat.

3. Das Verfahren nach Anspruch 2, bei dem der Patient als ein Responder auf die therapeutische Behandlung eingestuft wird, wenn das Verhältnis der Menge an HMW-Adiponektin zur Menge an Gesamt-Adiponektin oder LMW-Adiponektin nach dem Beginn der therapeutischen Behandlung um wenigstens 20% ansteigt.

4. Das Verfahren nach Anspruch 2, bei dem der Patient als ein Responder auf die therapeutische Behandlung eingestuft wird, wenn das Verhältnis der Menge an HMW-Adiponektin zur Menge an Gesamt-Adiponektin oder LMW-Adiponektin nach dem Beginn der therapeutischen Behandlung um wenigstens 25% ansteigt.

5. Das Verfahren nach Anspruch 2, bei dem der Patient als ein Responder auf die therapeutische Behandlung eingestuft wird, wenn das Verhältnis der Menge an HMW-Adiponektin zur Menge an Gesamt-Adiponektin oder LMW-Adiponektin nach dem Beginn der therapeutischen Behandlung um wenigstens 30% ansteigt.

6. Das Verfahren nach Anspruch 2, bei dem der Patient als ein Responder auf die therapeutische Behandlung eingestuft wird, wenn das Verhältnis der Menge an HMW-Adiponektin zur Menge an Gesamt-Adiponektin oder LMW-Adiponektin nach dem Beginn der therapeutischen Behandlung um wenigstens 40% ansteigt.

7. Das Verfahren nach Anspruch 2, bei dem der Patient als ein Responder auf die therapeutische Behandlung eingestuft wird, wenn das Verhältnis der Menge an HMW-Adiponektin zur Menge an Gesamt-Adiponektin oder LMW-Adiponektin nach dem Beginn der therapeutischen Behandlung um wenigstens 50% ansteigt.

8. Das Verfahren nach Anspruch 2, bei dem der Patient als ein Responder auf die therapeutische Behandlung eingestuft wird, wenn das Verhältnis der Menge an HMW-Adiponektin zur Menge an Gesamt-Adiponektin oder LMW-Adiponektin nach dem Beginn der therapeutischen Behandlung um wenigstens 75% ansteigt.

9. Das Verfahren nach Anspruch 2, bei dem die insulinsensibilisierenden Pharmazeutika ausgewählt sind aus der Gruppe, bestehend aus PPAR-gamma-Agonisten, PPAR-gamma-Teilagonisten und dualen PPAR-alpha-gamma-Agonisten.

10. Das Verfahren nach Anspruch 2, bei dem das insulinsensibilisierende Pharmazeutikum eine TZD-Gruppe in seiner Struktur enthält.

11. Das Verfahren nach Anspruch 2, bei dem das insulinsensibilisierende Pharmazeutikum ausgewählt ist aus der Gruppe, bestehend aus Pioglitazon, Rosiglitazon, MCC-555, Balaglitazon, Isaglitazon, Netoglitazon, KRP-297 (MK-0767), Farglitazar, Tesaglitazar (AZ-242) und Muraglitazar (BMS-298585).

12. Ein Verfahren gemäß einem vorhergehenden Anspruch, bei dem das Verhältnis der Menge an HMW-Adiponektin zur Menge an Gesamt-Adiponektin gemessen wird.

13. Das Verfahren nach Anspruch 2, bei dem der Patient als ein Responder auf die therapeutische Behandlung eingestuft wird, wenn das Verhältnis der Menge an HMW-Adiponektin zur Menge an Gesamt-Adiponektin in dem Plasma oder Serum des Patienten innerhalb von vier Wochen nach dem Beginn der therapeutischen Behandlung um wenigstens 20% ansteigt.

14. Das Verfahren nach Anspruch 13, bei dem der Patient als ein Responder auf die therapeutische Behandlung eingestuft wird, wenn das Verhältnis der Menge an HMW-Adiponektin zur Menge an Gesamt-Adiponektin in dem Plasma oder Serum des Patienten innerhalb von zwei Wochen nach dem Beginn der therapeutischen Behandlung um wenigstens 20% ansteigt.

15. Das Verfahren nach Anspruch 13, bei dem der Patient als ein Responder auf die therapeutische Behandlung eingestuft wird, wenn das Verhältnis der Menge an HMW-Adiponektin zur Menge an Gesamt-Adiponektin in dem Plasma oder Serum des Patienten innerhalb von einer Woche nach dem Beginn der therapeutischen Behandlung um wenigstens 20% ansteigt.

16. Ein Verfahren zur Vorhersage, ob eine therapeutische Behandlung gegen Insulinresistenz zur Besserung von einer oder mehreren Erkrankungen, die mit Insulinresistenz verbunden sind, bei einem Patienten, der eine Behandlung gegen die Erkrankung oder Erkrankungen benötigt, wirksam sein wird, umfassend die Schritte:
Messen der Menge an HMW-Adiponektin und der Menge an Gesamt-Adiponektin in Plasma oder Serum, das von dem Patienten vor dem Beginn der therapeutischen Behandlung erhalten wird, und
Messen der Menge an HMW-Adiponektin und der Menge an Gesamt-Adiponektin in Plasma oder Serum, das von dem Patienten ein- oder mehrmals nach dem Beginn der therapeutischen Behandlung erhalten wird,
wobei die therapeutische Behandlung die Verabreichung einer wirksamen Menge von einem oder mehreren insulinsensibilisierenden Pharmazeutika umfasst hat,
und wobei die therapeutische Behandlung als wirksam zur Besserung der einen oder mehreren Erkrankungen bei dem Patienten vorhergesagt wird, wenn das Verhältnis der Menge an HMW-Adiponektin zu der Menge an Gesamt-Adiponektin innerhalb von vier Wochen nach dem Beginn der therapeutischen Behandlung um wenigstens 20% ansteigt.

17. Das Verfahren gemäß Anspruch 16, wobei die eine oder mehreren Erkrankungen, die mit Insulinresistenz verbunden ist/sind, ausgewählt ist/sind aus der Gruppe, bestehend aus Typ-2-Diabetes, Fettsucht, Hypertonie und Dyslipidämie.

18. Das Verfahren nach Anspruch 16, wobei das Verfahren verwendet wird, um vorherzusagen, ob eine therapeutische Behandlung gegen Insulinresistenz zur Besserung von Hyperglykämie oder Dyslipidämie bei einem Typ-2-Diabetes-Patienten wirksam sein wird.

19. Das Verfahren nach Anspruch 16, wobei das Verfahren verwendet wird, um vorherzusagen, ob eine therapeutische Behandlung gegen Insulinresistenz zur Verringerung des Risikos, dass ein nichtdiabetischer Patient mit verminderter Glukosetoleranz oder erhöhter Nüchtemplasmaglukose Typ-2-Diabetes entwickeln wird, wirksam sein wird.

20. Das Verfahren nach Anspruch 16, wobei das Verfahren verwendet wird, um vorherzusagen, ob eine therapeutische Behandlung gegen Insulinresistenz zur Besserung von drei oder mehreren Symptomen des metabolischen Syndroms wirksam sein wird, wobei die Symptome ausgewählt sind aus der Gruppe, bestehend aus abdominaler Fettsucht, Hypertriglyceridämie, niedrigem HDL, Bluthochdruck und erhöhter Nüchternglukose.

21. Ein Verfahren gemäß einem der Ansprüche 18-20, bei dem die therapeutische Behandlung als wirksam vorhergesagt wird, wenn das Verhältnis der Menge an HMW-Adiponektin zur Menge an Gesamt-Adiponektin in dem Plasma oder Serum des Patienten innerhalb von vier Wochen nach dem Beginn der therapeutischen Behandlung um wenigstens 25% ansteigt.

22. Ein Verfahren gemäß einem der Ansprüche 18-20, bei dem die therapeutische Behandlung als wirksam vorhergesagt wird, wenn das Verhältnis der Menge an HMW-Adiponektin zur Menge an Gesamt-Adiponektin in dem Plasma oder Serum des Patienten innerhalb von vier Wochen nach dem Beginn der therapeutischen Behandlung um wenigstens 30% ansteigt.

23. Ein Verfahren gemäß einem der Ansprüche 18-20, bei dem die therapeutische Behandlung als wirksam vorhergesagt wird, wenn das Verhältnis der Menge an HMW-Adiponektin zur Menge an Gesamt-Adiponektin in dem Plasma oder Serum des Patienten innerhalb von vier Wochen nach dem Beginn der therapeutischen Behandlung um wenigstens 40% ansteigt.

24. Ein Verfahren gemäß einem der Ansprüche 18-20, bei dem die therapeutische Behandlung als wirksam vorhergesagt wird, wenn das Verhältnis der Menge an HMW-Adiponektin zur Menge an Gesamt-Adiponektin in dem Plasma oder Serum des Patienten innerhalb von vier Wochen nach dem Beginn der therapeutischen Behandlung um wenigstens 50% ansteigt.

25. Ein Verfahren gemäß einem der Ansprüche 18-20, bei dem die therapeutische Behandlung als wirksam vorhergesagt wird, wenn das Verhältnis der Menge an HMW-Adiponektin zur Menge an Gesamt-Adiponektin in dem Plasma oder Serum des Patienten innerhalb von vier Wochen nach dem Beginn der therapeutischen Behandlung um wenigstens 75% ansteigt.

## Revendications

1. Méthode permettant de déterminer si un patient insulino-résistant est un répondeur à un traitement thérapeutique pour l'insulino-résistance, comprenant les étapes consistant à :
Mesurer la quantité d'adiponectine-HMW et la quantité d'adiponectine totale ou d'adiponectine-LMW dans le plasma ou le sérum obtenu d'un patient avant le commencement dudit traitement thérapeutique; et
Mesurer la quantité d'adiponectine-HMW et la quantité d'adiponectine totale ou d'adiponectine-LMW dans le plasma ou le sérum obtenu dudit patient une fois ou plusieurs fois après le commencement dudit traitement thérapeutique,
dans laquelle il est déterminé que ledit patient est un répondeur audit traitement thérapeutique si le rapport de la quantité d'adiponectine-HMW à la quantité d'adiponectine totale ou d'adiponectine-LMW augmente après le commencement dudit traitement thérapeutique.

2. La méthode de la revendication 1, dans laquelle ledit traitement thérapeutique comprend l'administration d'une quantité efficace d'un ou de plusieurs produits pharmaceutiques de sensibilisation à l'insuline.

3. La méthode de la revendication 2, dans laquelle il est déterminé que ledit patient est un répondeur audit traitement thérapeutique si le rapport de la quantité d'adiponectine-HMW à la quantité d'adiponectine totale ou d'adiponectine-LMW augmente d'au moins 20% après le commencement dudit traitement thérapeutique.

4. La méthode de la revendication 2, dans laquelle il est déterminé que ledit patient est un répondeur audit traitement thérapeutique si le rapport de la quantité d'adiponectine-HMW à la quantité d'adiponectine totale ou d'adiponectine-LMW augmente d'au moins 25% après le commencement dudit traitement thérapeutique.

5. La méthode de la revendication 2, dans laquelle il est déterminé que ledit patient est un répondeur audit traitement thérapeutique si le rapport de la quantité d'adiponectine-HMW à la quantité d'adiponectine totale ou d'adiponectine-LMW augmente d'au moins 30% après le commencement dudit traitement thérapeutique.

6. La méthode de la revendication 2, dans laquelle il est déterminé que ledit patient est un répondeur audit traitement thérapeutique si le rapport de la quantité d'adiponectine-HMW à la quantité d'adiponectine totale ou d'adiponectine-LMW augmente d'au moins 40% après le commencement dudit traitement thérapeutique.

7. La méthode de la revendication 2, dans laquelle il est déterminé que ledit patient est un répondeur audit traitement thérapeutique si le rapport de la quantité d'adiponectine-HMW à la quantité d'adiponectine totale ou d'adiponectine-LMW augmente d'au moins 50% après le commencement dudit traitement thérapeutique.

8. La méthode de la revendication 2, dans laquelle il est déterminé que ledit patient est un répondeur audit traitement thérapeutique si le rapport de la quantité d'adiponectine-HMW à la quantité d'adiponectine totale ou d'adiponectine-LMW augmente d'au moins 75% après le commencement dudit traitement thérapeutique.

9. La méthode de la revendication 2, dans laquelle lesdits produits pharmaceutiques de sensibilisation à l'insuline sont sélectionnés parmi le groupe consistant en agonistes de PPAR-gamma, agonistes partiels de PPAR-gamma et co-agonistes de PPAR-alpha et gamma.

10. La méthode de la revendication 2, dans laquelle ledit produit pharmaceutique de sensibilisation à l'insuline a un groupe TZD dans sa structure.

11. La méthode de la revendication 2, dans laquelle ledit produit pharmaceutique de sensibilisation à l'insuline est sélectionné parmi le groupe consistant en pioglitazone, rosiglitazone, MCC-555, balaglitazone, isaglitazone, netoglitazone, KRP-297 (MK-0767), farglitazar, tesaglitazar (AZ-242) et muraglitazar (BMS-298585).

12. Méthode selon l'une quelconque des revendications précédentes, dans laquelle le rapport de la quantité d'adiponectine-HMW à la quantité d'adiponectine totale est mesuré.

13. La méthode de la revendication 2, dans laquelle il est déterminé que ledit patient est un répondeur audit traitement thérapeutique si le rapport de la quantité d'adiponectine-HMW à la quantité d'adiponectine totale dans le plasma ou le sérum dudit patient, augmente d'au moins 20% dans les quatre semaines qui suivent le commencement dudit traitement thérapeutique.

14. La méthode de la revendication 13, dans laquelle il est déterminé que ledit patient est un répondeur audit traitement thérapeutique si le rapport de la quantité d'adiponectine-HMW à la quantité d'adiponectine totale dans le plasma ou le sérum dudit patient, augmente d'au moins 20% dans les deux semaines qui suivent le commencement dudit traitement thérapeutique.

15. La méthode de la revendication 13, dans laquelle il est déterminé que ledit patient est un répondeur audit traitement thérapeutique si le rapport de la quantité d'adiponectine-HMW à la quantité d'adiponectine totale dans le plasma ou le sérum dudit patient, augmente d'au moins 20% dans la semaine qui suit le commencement dudit traitement thérapeutique.

16. Méthode permettant de prévoir si un traitement thérapeutique pour l'insulino-résistance sera efficace pour améliorer une ou plusieurs maladies associées à l'insulino-résistance chez un patient ayant besoin de traitement pour ladite ou lesdites maladies, comprenant les étapes consistant à :
Mesurer la quantité d'adiponectine-HMW et la quantité d'adiponectine totale dans le plasma ou le sérum obtenu dudit patient avant le commencement dudit traitement thérapeutique; et
Mesurer la quantité d'adiponectine-HMW et la quantité d'adiponectine totale dans le plasma ou le sérum obtenu dudit patient une fois ou plusieurs fois après le commencement dudit traitement thérapeutique;
dans laquelle ledit traitement thérapeutique comprend une quantité efficace d'un ou plusieurs produits pharmaceutiques de sensibilisation à l'insuline,
et dans laquelle ledit traitement thérapeutique est prévu être efficace pour améliorer ladite une ou lesdites plusieurs maladies chez ledit patient, si le rapport de la quantité d'adiponectine-HMW à la quantité d'adiponectine totale augmente d'au moins 20% dans les quatre semaines qui suivent le commencement dudit traitement thérapeutique.

17. La méthode selon la revendication 16, dans laquelle ladite une ou lesdites plusieurs maladies associées à l'insulino-résistance sont sélectionnées parmi le groupe consistant en diabète de type 2, obésité, hypertension et dyslipidémie.

18. La méthode de la revendication 16, dans laquelle ladite méthode est utilisée pour prévoir si un traitement thérapeutique pour l'insulino-résistance sera efficace pour améliorer l'hyperglycémie ou la dyslipidémie chez un patient diabétique de type 2.

19. La méthode de la revendication 16, dans laquelle ladite méthode est utilisée pour prévoir si un traitement thérapeutique pour l'insulino-résistance sera efficace pour réduire le risque qu'un patient non diabétique présentant une tolérance affaiblie au glucose ou un taux élevé de glucose plasmatique à jeun, développe un diabète de type 2.

20. La méthode de la revendication 16, dans laquelle ladite méthode est utilisée pour prévoir si un traitement thérapeutique pour l'insulino-résistance sera efficace pour améliorer trois symptômes du syndrome métabolique ou plus, lesdits symptômes étant sélectionnés parmi le groupe consistant en obésité abdominale, hypertriglycéridémie, faible taux de HDL, tension artérielle élevée et taux élevé de glucose à jeun.

21. Méthode selon l'une quelconque des revendications 18-20, dans laquelle ledit traitement thérapeutique est prévu être efficace si le rapport de la quantité d'adiponectine-HMW à la quantité d'adiponectine totale dans le plasma ou le sérum dudit patient, augmente d'au moins 25% dans les quatre semaines qui suivent le commencement dudit traitement thérapeutique.

22. Méthode selon l'une quelconque des revendications 18-20, dans laquelle ledit traitement thérapeutique est prévu être efficace si le rapport de la quantité d'adiponectine-HMW à la quantité d'adiponectine totale dans le plasma ou le sérum dudit patient, augmente d'au moins 30% dans les quatre semaines qui suivent le commencement dudit traitement thérapeutique.

23. Méthode selon l'une quelconque des revendications 18-20, dans laquelle ledit traitement thérapeutique est prévu être efficace si le rapport de la quantité d'adiponectine-HMW à la quantité d'adiponectine totale dans le plasma ou le sérum dudit patient, augmente d'au moins 40% dans les quatre semaines qui suivent le commencement dudit traitement thérapeutique.

24. Méthode selon l'une quelconque des revendications 18-20, dans laquelle ledit traitement thérapeutique est prévu être efficace si le rapport de la quantité d'adiponectine-HMW à la quantité d'adiponectine totale dans le plasma ou le sérum dudit patient, augmente d'au moins 50% dans les quatre semaines qui suivent le commencement dudit traitement thérapeutique.

25. Méthode selon l'une quelconque des revendications 18-20, dans laquelle ledit traitement thérapeutique est prévu être efficace si le rapport de la quantité d'adiponectine-HMW à la quantité d'adiponectine totale dans le plasma ou le sérum dudit patient, augmente d'au moins 75% dans les quatre semaines qui suivent le commencement dudit traitement thérapeutique.
